# NEUE EUROPÄISCHE PATENTSCHRIFT

(11) **EP 1 525 178 B2**
(45) Veröffentlichungstag und Bekanntmachung des Hinweises auf die Entscheidung über den Einspruch: **25.09.2013**
(45) Hinweis auf die Patenterteilung: 07.04.2010
(21) Anmeldenummer: 03764962.1
(22) Anmeldetag: 10.07.2003
(51) Int. Cl.: C07C 51/25, C07C 51/215, C07C 57/05, C07C 57/055, B01J 8/06, B01J 8/00

(54) **VERFAHREN DER HETEROGEN KATALYSIERTEN GASPHASEN-PARTIALOXIDATION WENIGSTENS EINER ORGANISCHEN VERBINDUNG**
METHOD FOR THE HETEROGENEOUSLY-CATALYSED GAS PHASE PARTIAL OXIDATION OF AT LEAST ONE ORGANIC COMPOUND
PROCEDE D'OXYDATION PARTIELLE CATALYTIQUE HETEROGENE EN PHASE GAZEUSE D'AU MOINS UN COMPOSE ORGANIQUE

(30) Priorität: 18.07.2002 DE 10232748
(43) Veröffentlichungstag der Anmeldung: 27.04.2005
(73) Patentinhaber: BASF SE, 67056 Ludwigshafen (DE)
(72) Erfinder: HAMMON, Ulrich, 68163 Mannheim (DE); PETZOLDT, Jochen, 68163 Mannheim (DE); MÜLLER-ENGEL, Klaus, Joachim, 76297 Stutensee (DE); DIETERLE, Martin, 68167 Mannheim (DE)
(86) Internationale Anmeldenummer: PCT/EP2003/007456
(87) Internationale Veröffentlichungsnummer: WO 2004/009525

(56) Entgegenhaltungen:
- EP-A- 0 614 872
- EP-A1- 0 987 057
- EP-A2- 1 226 865
- WO-A1-00/53557
- WO-A1-98/02239
- DE-A1- 10 232 967
- GB-A- 1 277 050
- JP-A- H0 892 147
- JP-A- H11 130 722
- US-A- 5 462 652
- US-A- 5 739 391
- US-A- 5 821 390
- TAM, P.S. ET AL: 'Band-Aging Catalyst Deactivation: Analytical Solutions' AICHE JOURNAL Bd. 32, Nr. 7, Juli 1986, Seiten 1205 - 1207
- ERTL,G , KNÖZINGER, H., WEITKAMP, J.: 'Handbook of Heterogeneous Catalysis', Bd. 3, 1997, VCH, WEINHEIM vol. EIGENBERGER, G.: 'Catalytic Fixed-Bed Reactors', Seiten 1399 - 1425
- SADHUKHAN, P. ET AL: 'Oxidation of Naphthalene in Packed-Bed Reactor with Catalyst Activity Profile: ADesign Scheme for Improved Reactor Stability and Higher Product Yield' AICHE JOURNAL Bd. 22, Nr. 4, Juli 1976, Seiten 808 - 810

## Beschreibung

Vorliegende Erfindung betrifft ein Verfahren der heterogen katalysierten Gasphasen-Partialoxidation wenigstens einer organischen Verbindung mit molekularem Sauerstoff an einem in einem Vielkontaktrohr-Festbettreaktor befindlichen Katalysatorfestbett, bei dem ab einem bestimmten Betriebszeitpunkt mit zunehmender Betriebsdauer eine zunehmende Minderung der Qualität der Katalysatorbeschickung einhergeht.

Unter einer vollständigen Oxidation einer organischen Verbindung mit molekularem Sauerstoff wird hier verstanden, daß die organische Verbindung unter reaktiver Einwirkung von molekularem Sauerstoff so umgesetzt wird, daß der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden hier als Partialoxidation einer organischen Verbindung zusammengefaßt.

Im besonderen sollen hier unter Partialoxidation solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält als vor Durchführung der Partialoxidation.

Es ist allgemein bekannt, daß durch partielle und heterogen katalysierte Oxidation verschiedener organischer Verbindungen mit molekularem Sauerstoff in der Gasphase zahlreiche Grundchemikalien erzeugt werden können. Beispielhaft genannt (vgl. auch EP-A 731080) seien die Umsetzung von Propylen zu Acrolein und/ oder Acrylsäure (vgl. z.B. DE-A 2351151), die Umsetzung von tert.-Butanol, iso-Buten, iso-Butan, iso-Butyraldehyd oder dem Methylether des tert.Butanols zu Methacrolein und/oder Methacrylsäure (vgl. z.B. DE-A 2526238, EP-A 92097, EP-A 58927,
DE-A 4132263, DE-A 4132684 und DE-A 4022212), die Umsetzung von Acrolein zu Acrylsäure, die Umsetzung von Methacrolein zu Methacrylsäure (vgl. z.B. DE-A 2526238), die Umsetzung von o-Xylol oder Naphthalin zu Phthalsäureanhydrid (vgl. z.B. EP-A 522871), die Umsetzung von Butadien zu Maleinsäureanhydrid (vgl. z.B. DE-A 2106796 und DE-A 1624921), die Umsetzung von n-Butan zu Maleinsäureanhydrid (vgl. z.B. GB-A, 1464198 und GB-A 1291354), die Umsetzung von Indanen zu z.B. Anthrachinon (vgl. z.B.
DE-A 2025430), die Umsetzung von Ethylen zu Ethylenoxid oder Propylen zu Propylenoxid (vgl. z.B. DE-AS 1254137, DE-A 2159346,
EP-A 372972, WO 89/0710, DE-A 4311608 und Beyer, Lehrbuch der organischen Chemie, 17. Auflage (1973), Hirzel Verlag Stuttgart, S. 261), die Umsetzung von Propylen und/oder Acrolein zu Acrylnitril (vgl. z.B. DE-A 2351151), die Umsetzung von iso-Buten und/oder Methacrolein zu Methacrylnitril (d.h., der Begriff der partiellen Oxidation soll in dieser Schrift auch den Begriff der partiellen Ammoxidation, d.h. eine partielle Oxidation im Beisein von Ammoniak, umfassen), die oxidative Dehydrierung von Kohlenwasserstoffen (vgl. z.B. DE-A 2351151), die Umsetzung von Propan zu Acrylnitril oder zu Acrolein und/oder Acrylsäure (vgl. z.B. DE-A 10131297, EP-A 1090684, EP-A 608838, DE-A 10046672, EP-A 529853, WO 01/96270 und DE-A 10028582) etc..

Um zu bewirken, daß die gewünschte Gasphasen-Partialoxidation gegenüber der vollständigen Oxidation bevorzugt abläuft, wird erstere üblicherweise als heterogen katalysierte Oxidation an der Oberfläche von im festen Zustand befindlichen Katalysatoren durchgeführt. Bei den Festkörperkatalysatoren handelt es sich häufig um Oxidmassen oder um Edelmetalle (z.B. Ag). Die katalytisch aktive Oxidmasse kann neben Sauerstoff lediglich ein anderes Element oder mehr als ein anderes Element (im Fall von sogenannten Multielementoxidmassen) enthalten.

Besonders häufig kommen als katalytisch aktive Oxidmassen solche zur Anwendung, die mehr als ein metallisches, insbesondere übergangsmetallisches, Element umfassen. In diesem Fall spricht man Multimetalloxidmassen. Üblicherweise sind Multielementoxidmassen keine einfachen physikalischen Gemische von Oxiden der elementaren Konstituenten, sondern heterogene Gemische von komplexen Polyverbindungen dieser Elemente. In der Praxis werden die vorgenanntcn katalytisch aktiven Festmassen in der Regel zu unterschiedlichsten Geometrien geformt (Ringe, Vollzylinder, Kugeln etc.) eingesetzt. Die Formung kann dabei so erfolgen, daß die katalytisch aktive Masse als solche (z.B. in Extrudern) geformt wird, so daß im Ergebnis ein sogenannter Vollkatalysator resultiert, oder dadurch, daß die Aktivmasse auf einen vorgeformten Träger aufgebracht wird. Beispiele für Katalysatoren die für heterogen katalysierte Gasphasen-Partialoxidationen geeignet sind finden sich z.B. in der DE-A 10046957, in der EP-A 1097745, in der DE-A 4431957, in der DE-A 10046928, in der DE-A 19910506, in der DE-A 19622331, in der DE-A 10121592, in der EP-A 700714, in der DE-A 19910508, in der EP-A 415347, in der EP-A 471853 und in der EP-A 700893).

Meist werden heterogen katalysierte Gasphasen-Partialoxidationen bei erhöhter Temperatur (in der Regel einige hundert °C, üblicherweise 100 bis 600°C) durchgeführt.

Der Arbeitsdruck kann bei heterogen katalysierten Gasphasen-Partialoxidationen unter 1 atm, bei 1 atm oder über 1 atm liegen. In der Regel liegt er im Bereich von 1 bis 10 atm.

Da die meisten heterogen katalysierten Gasphasen-Partialoxidationen stark exotherm verlaufen, ist infolge einer Vielfalt von möglichen Parallel- oder Folgereaktionen im Hinblick auf eine möglichst selektive Umsetzung der zu oxidierenden organischen Verbindung zum Zielprodukt die alleinige Maßnahme einer Katalysatormitverwendung nicht ausreichend. Vielmehr ist es für eine beherrschbare Durchführung von selektiven heterogen katalysierten Gasphasen-Partialoxidationen zusätzlich erforderlich, den Verlauf der Reaktionstemperatur in einem gewissen Umfang zu steuern.

Eine allgemein angewandte Möglichkeit der Lenkung der freiwerdenden Reaktionswärme besteht darin, die Reaktionspartner molekularer Sauerstoff und partiell zu oxidierende organische Verbindung mit Inertgasen wie N₂, Kohlenoxiden wie CO₂ und CO, inerten Kohlenwasserstoffen, rückgeführten Reaktionsabgasen und/oder Wasserdampf zu verdünnen, wobei die Verwendung von Verdünngungsgasen mit möglichst hoher molarer Wärmekapazität besonders vorteilhaft ist (vgl. EP-A 253409). Der Begriff inert meint dabei, daß solche Verdünnungsgase bevorzugt sind, die im Verlauf der Partialoxidation wenigstens zu 95 mol-%, bevorzugt zu wenigstens 97 mol-% oder 99 mol-% chemisch unverändert erhalten bleiben. Das Beschickungsgasgemisch einer heterogen katalysierten Gasphasen-Partialoxidation wenigstens einer organischen Verbindung wird daher neben dieser organischen Verbindung und molekularem Sauerstoff in der Regel zusätzlich wenigstens ein inertes Verdünnungsgas umfassen.

Eine weitere generell angewandte Methode zur Steuerung der Reaktionstemperatur besteht darin, die heterogen katalysierte Gasphasen-Partialoxidation in einem Vielkontaktrohr-Festbettreaktor durchzuführen. Solche Reaktoren entsprechen in ihrem Typ den Mantel-Rohr-Wärmeaustauschem. D.h., ihre übliche Bauart besteht aus einem, in der Regel zylinderförmigen, Behälter, in welchem eine Vielzahl von Rohren (ein Rohrbündel) entsprechend den Kühlrohren eines Mantel-Rohr-Wärmeaustauschers in üblicherweise vertikaler Anordnung untergebracht ist. Diese Kontaktrohre, von denen ein jedes eine Festbettanordnung der entsprechenden Katalysatorbeschickung enthält, sind mit ihren Enden in Rohrböden abdichtend befestigt und münden in je eine am oberen bzw. unteren Ende mit dem Behälter verbundene Haube. Über diese Hauben wird das die Kontaktrohre durchströmende Reaktionsgasgemisch zu- bzw. abgeführt, so daß jedes Kontaktrohr einer langgestreckten Reaktionseinheitszone entspricht.

Die chemische Umsetzung erfolgt, wenn das Reaktionsgasgemisch das Festbett durchströmt während der Verweilzeit des Reaktionsgasgemisches in selbigem.

Ferner werden durch den die Kontaktrohre umgebenden Raum Wärmeaustauschmittel geleitet, um die Prozeßwärme zu bewältigen. Nach dem Austritt aus dem Behälter werden die Wärmeaustauschmittel, z.B. in äußeren Wärmetauschern, wieder auf ihre ursprüngliche Temperatur gebracht, bevor sie wieder in den Reaktionsbehälter eintreten (vgl. z.B. DE-A 30242468).

Tritt längs der Kontaktrohre auf unterschiedlichen (mehreren) Höhen Wärmeaustauschmittel in den Reaktor ein, so soll hier von einer Anwendung mehrerer Wärmeaustauschmittelkreisläufe oder auch von einem mehrere Temperierzonen aufweisenden Mehrzonenreaktor gesprochen werden (die einzelnen Kreisläufe sind durch geeignete Trennbleche in der Regel weitgehend voneinander getrennt). Erfolgt der Eintritt des Wärmeaustauschmittels lediglich auf einer Höhe, wird hier von einem Wärmeaustauschmittelkreislauf oder auch von einem Einzonenreaktor gesprochen, selbst wenn dieser Kreislauf nicht mit einer, sondern aus Gründen der Zweckmäßigkeit mit mehreren Pumpen betrieben wird.

Beispiele für solche Einzonen- und Mehrzonenreaktoren finden sich z.B. in den Schriften DE-A 10024348, DE-A 19836792, DE-A 10032304, WO 01/87476, DE-A 19910508, DE-A 19910506, DE-A 19927624, DE-A 19948241, DE-A 19948248, DE-A 19948523, DE-A 1995516, DE-A 10134026, DE-A 10101695, US-A 5442108, EP-A 911313, EP-A 1097745, DE-A 10137768, DE-A 10135498 und DE-A 10040781.

Üblicherweise sind die Kontaktrohre aus ferritischem Stahl gefertigt und weisen häufig eine Wanddicke von 1 bis 3 mm auf. Ihr Innendurchmesser beträgt vielfach 20 bis 30 mm. Die Rohrlänge erstreckt sich im Normalfall auf wenige Meter (typisch ist eine Kontaktrohrlänge im Bereich von 2 bis 4 m). Anwendungstechnisch zweckmäßig beläuft sich die im Behälter untergebrachte Anzahl von Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, daß der Abstand der zentrischen Innenachsen von zueinander nächstliegenden Kontaktrohren (die sogenannte Kontaktrohrteilung) 30 bis 50 mm, häufig 35 bis 45 mm beträgt (vgl. z.B. EP-A 468290).

Als Wärmeaustauschmittel eignen sich insbesondere fluide Temperiermedien. Häufig ist die Verwendung von Salzschmelzen, z.B. die von Salzen wie Kaliumnitrat, Kaliumnitrit, Natriumnitrit und/oder Natriumnitrat, geeignet. Teilweise werden aber auch Schmelzen von niedrig schmelzenden Metallen wie Natrium, Quecksilber sowie von Legierungen verschiedener Metalle verwendet.

Das Wärmeaustauschmittel kann dabei in einfacher Weise im wesentlichen direkt längs (im Gleichstrom oder im Gegenstrom zum Reaktionsgasgemisch) zu den Kontaktrohren geführt werden. Es besteht aber auch die Möglichkeit, diese Längsführung (im Gleichstrom oder im Gegenstrom zum Reaktionsgasgemisch) lediglich über den gesamten Reaktionsbehälter betrachtet zu verwirklichen und dieser Längsströmung innerhalb des Reaktionsbehälters durch eine längs der Kontaktrohre aufeinanderfolgende Anordnung von Durchtrittsquerschnitte freilassenden Umlenksscheiben, eine Querströmung so zu überlagern, daß im Längsschnitt durch das Rohrbündel ein mäanderförmiger Strömungsverlauf des Wärmeaustauschmittels resultiert. In der Regel verläßt das Wärmeaustauschmittel den Reaktor mit einer Temperatur, die oberhalb seiner Eintrittstemperatur liegt. Vorstehende Aussagen besitzen insbesondere Gültigkeit für die heterogen katalysierte Gasphasen-Partialoxidation von Propylen zu Acrolein und/oder Acrylsäure, von iso-Buten zu Methacrolein und/oder Methacrylsäure, von (Meth)acrolein zu (Meth) acrylsäure, von Propan zu Acrolein und/oder Acrylsäure sowie von iso-Butan zu Methacrolein und/oder Methacrylsäure.

Nachteilig an Verfahren der heterogen katalysierten Gasphasen-Partialoxidation wenigstens einer organischen Verbindung mit molekularem Sauerstoff an in einem Vielkontaktrohr-Festbettreaktor befindlichen Katalysatorfestbett ist, daß ab einem bestimmten Betriebszeitpunkt mit zunehmender Betriebsdauer eine zunehmende Minderung der Qualität der Katalysatorbeschickung eintritt. Diese betrifft in der Regel sowohl die Katalysatoraktivität (je höher die für einen bestimmten Umsatz erforderliche Temperatur, desto niedriger ist die Aktivität) als auch die Selektivität der Zielproduktbildung.

Es ist auch bekannt, daß die Reaktionstemperatur in Richtung der Strömung des Reaktionsgasgemisches längs eines Kontaktrohres in der Regel durch ein gewisses Maximum läuft (vgl. z.B. DE-A 4431949), was die Katalysatorqualität in diesem Bereich mit der Zeit teilweise verstärkt mindert. Zur Überwindung des genannten Nachteils werden im Stand der Technik unterschiedlichste Empfehlungen gegeben. Ein Vorschlag besteht in der Verkleinerung des Durchmessers der Kontaktrohre, um so die Wärmeableitung je Volumeneinheit des Katalysators zu erhöhen. Nach einem anderen vorgeschlagenen Verfahren wird die Ausbildung der Maxima dadurch zu unterdrücken versucht, daß man die volumenspezifische Aktivität der katalytischen Beschickung längs der Kontaktrohre variiert (z.B. in Strömungsrichtung stufenförmig oder kontinuierlich zunehmend gestaltet). Eine alternative Möglichkeit zur Minderung der Maximaausbildung besteht darin, die Belastung des Reaktors mit Reaktionsgasgemisch zu senken oder das Salzbad (Wärmeaustauschmittel) mäanderförmig fließen zu lassen, um die Wärmeabfuhr zu verbessern. Teilweise wird auch ein Rohrbündelreaktor mit mehr als einer Temperaturzone empfohlen, wobei die Temperatur der ersten Zone in Strömungsrichtung besonders niedrig, in der Regel niedriger als in den nachfolgenden Stufen, gewählt wird. Die genannten Maßnahmen können jedoch nicht verhindern, daß sich mit zunehmender Betriebsdauer die Qualtität der gesamten Katalysatorbeschickung per se verringert, und dies auch dann, wenn vorgenannte Maßnahmen zur Ausbildung der Maxima angewendet werden.

Dieser Tatsache versucht man im Stand der Technik dadurch zu begegnen, daß man die Katalysatorbeschickung durch Überleiten gecigneter, molekularen Sauerstoff enthaltender, Regeneriergase nach gewissen Betriebsdauern von Zeit zu Zeit regeneriert (vgl. EP-A 614872). Nachteilig an dieser Verfahrensweise ist jedoch, daß sich ihre Wirksamkeit mit zunehmender Gesamtbetriebsdauer erschöpft.

Im übrigen begegnet man im Stand der Technik der Erschöpfung der Qualität der Katalysatorbeschickung mit zunehmender Betriebsdauer dadurch, daß die gesamte Katalysatorbeschickung nach einer bestimmten Betriebsdauer aus dem Vielkontaktrohr-Festbettreaktor entnommen und durch eine frische Katalysatorbeschickung ersetzt wird.

Nachteilig in dieser Verfahrensweise ist jedoch, daß die Herstellung der benötigten Katalysatoren vergleichsweise aufwendig und teuer ist. In der Regel bewegen sich die Kosten für eine vollständige Katalysatorbeschickung eines großtechnischen Viel-kontaktrohr-Festbettreaktors im siebenstelligen Bereich.

Die Aufgabe der vorliegenden Erfindung bestand daher darin, ein verbessertes Verfahren der heterogen katalysierten Gasphasen-Partialoxidation wenigstens einer organischen Verbindung mit molekularem Sauerstoff an einem in einem Vielkontaktrohr-Festbettreaktor befindlichen Katalysatorfestbett, bei dem ab einem bestimmten Betriebszeitpunkt mit zunehmender Betriebsdauer eine zunehmende Minderung der Qualität der Katalysatorbeschickung einhergeht, zur Verfügung zu stellen, das der beschriebenen Katalysatorerschöpfung in besserer Weise Rechnung trägt als die geschilderten Maßnahmen des Standes der Technik.

Demgemäß wurde ein Verfahren der heterogen katalysierten Gasphasen-Partialoxidation wenigstens einer organischen Verbindung mit molekularem Sauerstoff an einem in einem Vielkontaktrohr-Festbettreaktor befindlichen Katalysatorfestbett, bei dem ab einem bestimmten Betriebszeitpunkt mit zunehmender Betriebsdauer eine zunehmende Minderung der Qualität der Katalysatorbeschickung einhergeht, gefunden, das dadurch gekennzeichnet ist, daß zur Rückgewinnung der Qualität der Katalysatorbeschickung nicht die gesamte gebrauchte Katalysatorbeschickung sondern nur eine Teilmenge derselben aus dem Vielkontaktrohr-Festbettreaktor entnommen und durch eine frische Katalysatorbeschickung ersetzt wird, und
- die Gasphasen-Partialoxidation diejenige von Acrolein zu Acrylsäure ist;
- die Volumenspezifische Aktivität der Katalysatorbeschichtung in Strömungsrichtung des Reaktionsgasgemischs kontinuierlich oder stufenförmig zunimmt; und
- sich der Teilkatalysatorwechsel in Strömungsrichtung auf 30 bis 50% der Schüttlange des Katalysatorfestbetts erstreckt.

In überraschender Weise bedingt die vorgenannte Maßnahme auch dann eine überproportionale Wiederherstellung der Performance der Katalysatorbeschickung, wenn Maßnahmen ergriffen wurden (z.B. die vorstehend genannten wie in Strömungsrichtung des Beschickungsgasgemisches längs der Kontaktrohre stufenweise oder kontinuierlich zunehmende volumenspezifische Aktivität der Katalysatorbeschickung und/oder mäanderförmiger Fluß des Wärmeaustauschmediums und/oder zwei Temperaturzonen, von denen die erste in Strömungsrichtung des Reaktionsgases eine tiefere Temperatur aufweist als die zweite), die die Ausbildung eines Maximums der Reaktionstemperatur längs des Kontaktrohres weitgehend unterdrücken.

Dies wird häufig darauf zurückgeführt, daß das Beschickungsgasgemisch in der Regel in geringen Mengen entweder bereits Katalysatorgifte enthält, weil die großtechnische Beschickung nicht von hochreinen Rohstoffen ausgeht, oder daß sich solche Katalysatorgifte erst im Verlauf der durchgeführten Partialoxidation bilden. Bezüglich solcher Katalysatorgifte wirkt das Katalysatorfestbett dann faktisch wie ein Absorber, in welchem sich das Katalysatorgift längs der Beschickung inhomogen anreichert.

Die erfindungsgemäße Verfahrensweise ist deshalb in der Regel in der zweiten Stufe einer zweistufigen heterogen katalysierten Gas-phasen-Partialoxidation von Propylen zu Acrylsäure besonders vorteilhaft, bei der das Produktgasgemisch aus der ersten Stufe ohne Zwischenreinigung, gegebenenfalls nach Zusatz von zusätzlichem für die Oxidation in der zweiten Stufe benötigtem molekularem Sauerstoff und gegebenenfalls zusätzlichem Inertgas, zur Beschickung des Vielkontaktrohr-Festbettreaktors der zweiten Oxidationsstufe verwendet wird.

In der ersten Stufe wird der Roh-Stoff zum Acrolein und in der zweiten Stufe das Acrolein zu Acrylsäure oxidiert.

Der beschriebene Zusammenhang trifft aber auch dann zu, wenn die Gesamtheit aller Schritt längs einer in einem einzigen Rohrbündelreaktor untergebrachten Katalysator-beschickung erfolgt, wie es z.B. die DE-A 10121592 am Beispiel der Gasphasen-Partialoxidation von Propylen zu Acrylsäure beschreibt.

Das als Rohstoff eingesetzte Propylen ist, wie bereits erwähnt, kein reiner Ausgangsstoff, sondern enthält stets gewisse Anteile an Verunreinigungen.

Beispielsweise wird als Propylen für die Acrylsäureherstellung häufig ein Roh-Propylen der beiden nachfolgenden Reinheitsgrade verwendet, wie sie aus Crackern abgetrennt werden:
a) Polymer grade Propylen:
   - ≥ 99,6 Gew.-%: Propen,
   - ≤ 0,4 Gew.-%: Propan,
   - ≤ 300 Gew.ppm: Ethan und/oder Methan,
   - ≤ 5 Gew.ppm: C₄-Kohlenwasserstoffe,
   - ≤ 1 Gew.ppm: Acetylen,
   - ≤ 7 Gew.ppm: Ethylen,
   - ≤ 5 Gew.ppm: Wasser,
   - ≤ 2 Gew.ppm: O₂,
   - ≤ 2 Gew.ppm: Schwefel enthaltende Verbindungen (berechnet als Schwefel),
   - ≤ 1 Gew.ppm: Chlor enthaltende Verbindungen (berechnet als Chlor),
   - ≤ 5 Gew.ppm: CO,
   - ≤ 5 Gew.ppm: CO₂,
   - ≤ 10 Gew.ppm: Cyclopropan,
   - ≤ 5 Gew.ppm: Propadien und/oder Propin,
   - ≤ 10 Gew.ppm: C_{≥5}-Kohlenwasserstoff und
   - ≤ 10 Gew.ppm: Carbonylgruppen enthaltende Verbindungen (berechnet als Ni(CO)₄);
b) Chemical grade Propylen:
   - ≥ 94 Gew.-%: Propen,
   - ≤ 6 Gew.-%: Propan,
   - ≤ 0,2 Gew.-%: Methan und/oder Ethan,
   - ≤ 5 Gew.ppm: Ethylen,
   - ≤ 1 Gew.ppm: Acetylen,
   - ≤ 20 Gew.ppm: Propadien und/oder Propin,
   - ≤ 100 Gew.ppm: Cyclopropan,
   - ≤ 50 Gew.ppm: Buten,
   - ≤ 50 Gew.ppm: Butadien,
   - ≤ 200 Gew.ppm: C₄-Kohlenwasserstoffe,
   - ≤ 10 Gew.ppm: C_{≥5}-Kohlenwasserstoffe,
   - ≤ 2 Gew.ppm: Schwefel enthaltende Verbindungen (berechnet als Schwefel),
   - ≤ 0,1 Gew.ppm: Sulfide (berechnet als H₂S),
   - ≤ 1 Gew.ppm: Chlor enthaltende Verbindungen (berechnet als Chlor),
   - ≤ 1 Gew.ppm: Chloride (berechnet als Cl^{θ}) und
   - ≤ 30 Gew.ppm: Wasser.

Selbstverständlich kann als Roh-Propylen auch ein im wesentlichen aus Propen und Propan bestehendes Gemisch verwendet werden, wie es die WO 01/96270 beschreibt.

Für den ersten Schritt, die heterogen katalysierte Gasphasen-Partialoxidation von Propylen zu Acrolein, kommen prinzipiell alle Mo, Bi und Fe enthaltenden Multimetalloxidmassen in Betracht.

Dies sind insbesondere die Multimetalloxidaktivmassen der allgemeinen Formel I der DE-A 19955176, die Multimetalloxidaktivmassen der allgemeinen Formel I der DE-A 19948523, die Multimetalloxidaktivmassen der allgemeinen Formel I der DE-A 19948523, die Multimetalloxidaktivmassen der allgemeinen Formeln I, II und III der DE-A 10101695, die Multimetalloxidaktivmassen der allgemeinen Formeln I, II und III der DE-A 19948248 und die Multimetalloxidaktivmassen der allgemeinen Formeln I, II und III der DE-A 19955163 sowie die in der EP-A 700714 genannten Multimetalloxidaktivmassen.

Ferner eignen sich für diesen Oxidationsschritt die Mo, Bi und Fe enthaltenden Multimetalloxidkatalysatoren die in den Schriften DE-A 10046957, DE-A 10063162, DE-C 3338380, DE-A 19902562, EP-A 15565, DE-C 2380765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1c aus der EP-A 15565 sowie ein in entsprechender Weise herzustellender Katalysator, dessen Aktivmasse jedoch die Zusammensetzung Mo₁₂Ni_{6,5}Zn₂Fe₂Bi₁P_{0,0065}K_{0,06}Oₓ·10SiO₂ aufweist. Ferner sind hervorzuheben das Beispiel mit der laufenden Nr. 3 aus der DE-A 19855913 (Stöchiometrie: Mo₁₂Co₇Fe₃Bi_{0,6}K_{0,08}Si_{1,6}Oₓ) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen.

Eine Vielzahl der für den Schritt vom Propylen zum Acrolein geeigneten Multimetalloxidaktivmassen läßt sich unter der allgemeinen Formel I 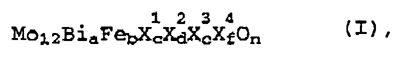 in der die Variablen nachfolgende Bedeutung aufweisen:
- X¹ =: Nickel und/oder Kobalt,
- X² =: Thallium, ein Alkalimetall und/oder ein Erdalkalimetall,
- X³ =: Zink, Phosphor, Arsen, Bor, Antimon, Zinn, Cer, Blei und/ oder Wolfram,
- X⁴ =: Silicium, Aluminium, Titan und/oder Zirkonium,

- a =: 0,5 bis 5,
- b =: 0,01 bis 5, vorzugsweise 2 bis 4,
- c =: 0 bis 10, vorzugsweise 3 bis 10,
- d =: 0 bis 2, vorzugsweise 0,02 bis 2,
- e =: 0 bis 8, vorzugsweise 0 bis 5,
- f =: 0 bis 10 und
- n =: eine Zahl, die durch die Wertigkeit und Häufigkeit der von Sauerstoff verschiedenen Elemente in I bestimmt wird,
subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden. Prinzipiell können Aktivmassen der allgemeinen Formel I in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate. Oxalate, Citrate, Acetate, Carbonate, Aminkomplexe, Ammonium-Salze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. üblicherweise werden dabei die Ausgangsverwindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die Multimetalloxidaktivmassen der allgemeinen Formel I können für den Schritt "Propen → Acrolein" sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/ oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, zirkondioxid, Siliciumcarbid oder silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl. EP-A 714 700).

Für den Schritt vom Propylen zum Acrolein zu verwendende Multimetalloxidaktivmassen sind ferner Massen der allgemeinen Formel II

[Y¹_{a'}Y²_{b'}O_{x'}]ₚ[Y³_{c'}Y⁴_{d'}Y⁵_{e'}Y⁶_{f'}Y⁷_{g'}Y²_{h'}O_{y'}]_{q} (II),

in der die Variablen folgende Bedeutung haben:
- Y² =: nur Wismut oder Wismut und wenigstens eines der Elemente Tellur, Antimon, Zinn und Kupfer,
- Y² =: Molybdän oder Molybdän und Wolfram,
- Y³ =: ein Alkalimetall, Thallium und/oder Samarium,
- Y⁴ =: ein Erdalkalimetall, Nickel, Kobalt, Kupfer, Mangan, Zink, Zinn, Cadmium und/oder Quecksilber,
- Y⁵ =: Eisen oder Eisen und wenigstens eines der Elemente Chrom und Cer,
- Y⁶ =: Phosphor, Arsen, Bor und/oder Antimon,
- Y⁷ =: ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

- a': = 0,01 bis 8,
- b': = 0,1 bis 30,
- c': = 0 bis 4,
- d': = 0 bis 20,
- e': > 0 bis 20,
- f': = 0 bis 6,
- g': = 0 bis 15,
- h': = 8 bis 16,
- x',y': = Zahlen, die durch die Wertigkeit und Häufigkeit der von Sauerstoff verschiedenen Elemente in II bestimmt werden und
- p,q: = Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'}, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte erfindungsgemäße Multimetalloxidmassen II sind solche, in denen Y¹ nur Wismut ist.

Unter diesen werden wiederum jene bevorzugt, die der allgemeinen Formel III

[Bi_{a"}Z²_{b"}O_{x"}]_{p"} [Z²₁₂Z³_{c"}Z⁴_{d"}Fe_{e"}Z⁵_{f"}Z⁶_{g"}Z⁷_{h"}O_{y"}]_{q"} (III)

in der die Varianten folgende Bedeutung haben:
- Z² =: Molybdän oder Molybdän und Wolfram,
- Z³ =: Nickel und/oder Kobalt,
- Z⁴ =: Thallium, ein Alkalimetall und/oder ein Erdalkalimetall,
- Z⁵ =: Phosphor, Arsen, Bor, Antimon, Zinn, Cer und/oder Blei,
- Z⁶ =: Silicium, Aluminium, Titan und/oder Zirkonium,
- Z⁷ =: Kupfer, Silber und/oder Gold,

- a" =: 0,1 bis 1,
- b" =: 0,2 bis 2,
- c" =: 3 bis 10,
- d" =: 0,02 bis 2,
- e" =: 0,01 bis 5, vorzugsweise 0,1 bis 3,
- f" =: 0 bis 5,
- g" =: 0 bis 10,
- h" =: 0 bis 1,
- x",y"=: Zahlen, die durch die Wertigkeit und Häufigkeit der von Sauerstoff verschiedenen Elemente in III bestimmt werden,
- p",q"=: Zahlen, deren Verhältnis p"/q" 0,1 bis 5, vorzugsweise 0,5 bis 2 beträgt,
entsprechen, wobei diejenigen Massen III ganz besonders bevorzugt werden, in denen Z²_{b"} = (Wolfram)_{b"} und Z²₁₂ = (Molybdän)₁₂ ist.

Ferner ist es von Vorteil, wenn wenigstens 25 mol-% (bevorzugt wenigstens 50 mol-% und besonders bevorzugt wenigstens 100 mol-%) des gesamten Anteils [Y¹_{a'}Y²_{b'}O_{x'}]ₚ ([Bi_{a"}Z²_{b"}O_{x"}]_{p"}) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusmmensetzung Y¹_{a'}Y²_{b'}Oₓ, [Bi_{a"}Z²_{b"}O_{x"}) vorliegen, deren Größtdurchmesser im Bereich 1 nm bis 100 µm liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der EP-A 575897 sowie in der DE-A 19855913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende Vorschüttung in Betracht.

Für den zweiten Schritt, die heterogen katalysierte Gasphasen-Partialoxidation von Acrolein zu Acrylsäure, kommen prinzipiell alle Mo und V enthaltenden Multimetalloxidmassen als Aktivmassen in Betracht, z.B. jene der DE-A 10046928.

Eine Vielzahl derselben, z.B. diejenigen der DE-A 19815281, läßt sich unter der allgemeinen Formel IV

Mo₁₂VₐX¹_{b}X²ₑX³_{d}X⁴ₑX⁵_{f}X⁶_{g}Oₙ (IV),

in der die Variablen folgende Bedeutung haben:
- X¹ =: W, Nb, Ta, Cr und/oder Ce,
- X² =: Cu, Ni, Co, Fe, Mn und/oder Zn,
- X³ =: Sb und/oder Bi,
- X⁴ =: eines oder mehrere Alkalimetalle,
- X⁵ =: eines oder mehrere Erdalkalimetalle,
- X⁶ =: Si, Al, Ti und/oder Zr,
- a =: 1 bis 6,
- b =: 0,2 bis 4,
- c =: 0,5 bis 18,
- d =: 0 bis 40,
- e =: 0 bis 2,
- f =: 0 bis 4,
- g =: 0 bis 40 und
- n =: eine Zahl, die durch die Wertigkeit und Häufigkeit der von Sauerstoff verschiedenen Elemente in IV bestimmt wird,
subsummieren.

Erfindungsgemäß bevorzugte Ausführungsformen innerhalb der aktiven Multimetalloxide IV sind jene, die von den nachfolgenden Bedeutungen der Variablen der allgemeinen Formel IV erfaßt werden:
- X¹ =: W, Nb, und/oder Cr,
- X² =: Cu, Ni, Co, und/oder Fe,
- X³ =: Sb,
- X⁴ =: Na und/oder K,
- X⁵ =: Ca, Sr und/oder Ba,
- X⁶ =: si, Al, und/oder Ti,
- a =: 1,5 bis 5,
- b =: 0,5 bis 2,
- c =: 0,5 bis 3,
- d =: 0 bis 2,
- e =: 0 bis 0,2,
- f =: 0 bis 1 und
- n =: eine Zahl, die durch die Wertigkeit und Häufigkeit der von Sauerstoff verschiedenen Elemente in IV bestimmt wird.

Erfindungsgemäß ganz besonders bevorzugte Multimetalloxide IV sind jedoch jene der allgemeinen Formel V

Mo₁₂V_{a'}Y¹_{b'}Y²_{c'}Y⁵_{f'}Y⁶_{g'}O_{n'} (V)

mit
- Y¹ =: W und/oder Nb,
- Y² =: Cu und/oder Ni,
- Y⁵ =: Ca und/oder Sr,
- Y⁶ =: Si und/oder Al,
- a' =: 2 bis 4,
- b' =: 1 bis 1,5,
- c' =: 1 bis 3,
- f' =: 0 bis 0,5,
- g' =: 0 bis 8 und
- n' =: eine Zahl, die durch die Wertigkeit und Häufigkeit der von Sauerstoff verschiedenen Elementen in V bestimmt wird.

Die erfindungsgemäß geeigneten Multimetalloxidaktivmassen (IV) sind in an sich bekannter, z.B. in der DE-A 4335973 oder in der EP-A 714700 offenbarter, Weise erhältlich.

Prinzipiell können für den Schritt "Acrolein → Acrylsäure" geeignete Multimetalloxidaktivmassen, insbesondere solche der allgemeinen Formel IV, in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wäßrigen Lösung und/oder Suspension miteinander vermischt. Besonders innige Trockengemisch werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die resultierenden Multimetalloxidmassen, insbesondere jene der allgemeinen Formel IV, können für die Acroleinoxidation sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/ oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl. EP-A 714 700).

Günstige für den Schritt "Acrolein → Acrylsäure" zu verwendende Multimetalloxidaktivmassen sind ferner Massen der allgemeinen Formel VI,

[D]ₚ[E]_{q} (VI),

in der die Variablen folgende Bedeutung haben:
- D =: Mo₁₂V_{a"}Z¹_{b"}Z²_{c"}Z³_{d"}Z⁴_{e"}Z⁵_{f"}Z⁶_{g"}O_{x"},
- E =: Z⁷₁₂Cu_{h"}H_{i"}O_{y"},
- Z¹ =: W, Nb, Ta, Cr und/oder Ce,
- Z² =: Cu, Ni, Co, Fe, Mn und/oder Zn,
- Z³ =: Sb und/oder Bi,
- Z⁴ =: Li, Na, K, Rb, Cs und/oder H
- Z⁵ =: Mg, Ca, Sr und/oder Ba,
- Z⁶ =: Si, Al, Ti und/oder Zr,
- Z⁷ =: Mo, W, V, Nb und/oder Ta, vorzugsweise Mo und/oder W

- a" =: 1 bis 8,
- b" =: 0,2 bis 5,
- c" =: 0 bis 23,
- d" =: 0 bis 50,
- e" =: 0 bis 2,
- f" =: 0 bis 5,
- g" =: 0 bis 50,
- h" =: 4 bis 30,
- i" =: 0 bis 20 und
- x",y" =: Zahlen, die durch die Wertigkeit und Häufigkeit der von Sauerstoff verschiedenen Elemente in VI bestimmt werden und
- p,q =: von Null verschiedene Zahlen, deren Verhältnis p/q 160:1 bis 1:1 beträgt,
und die dadurch erhältlich sind, daß man eine Multimetalloxid-masse E

Z⁷₁₂Cu_{h"}H_{i"}O_{y"} (E),

in feinteiliger Form getrennt vorbildet (Ausgangsmasse 1) und anschließend die vorgebildete feste Ausgrzngsmasse 1 in eine wäßrige Lösung, eine wäßrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D

Mo₁₂V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wäßrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen VI, bei denen die Einarbeitung der vorgebildeten festen Ausgangsmasse 1 in eine wäßrige Ausgangsmasse 2 bei einer Temperatur ≤ 70°C erfolgt. Eine detaillierte Beschreibung der Herstellung von Multimetalloxidmassen VI-Katalysatoren enthalten z.B. die EP-A 668104, die DE-A 19736105, die DE-A 10046928, die DE-A 19740493 und die DE-A 19528646.

Hinsichtlich der Formgebung gilt bezüglich Multimetalloxidmassen VI-Katalysatoren das bei den Multimetalloxidmassen IV-Katalysatoren Gesagte.

Für den Schritt "Acrolein → Acrylsäure" in hervorragender Weise geeignete Multimetallaxidkatalysatoren sind auch jene der DE-A 19815281, insbesondere mit Multimetaloxidaktivmassen der allgemeinen Formel I dieser Schrift.

Vorteilhaft werden für den Schritt vom Propylen zum Acrolein Vollkatalysatorxinge und für den Schritt vom Acrolein zur Acrylsäure Schalenkatalysatorringe verwendet.

Die Durchführung des ersten Schrittes, vom Propylen zum Acrolein, kann mit den beschriebenen Katalysatoren z. B. in einem Einzonen-Vielkontaktrohr-Festbettreaktor durchgeführt werden, wie ihn die DE-A 4431957 beschreibt.

Als Oxidationsmittel wird Sauerstoff verwendet. Wird als inertes Verdünnungsgas N₂ gewählt, erweist sich die Verwendung von Luft als Sauerstoffquelle besonders vorteilhaft.

In der Regel wird bei einem Propan : Sauerstoff : indifferente Gase (einschließlich Wasserdampf) Volumen (N1)-Verhältnis von 1:(1,0 bis 3,0) : (5 bis 25), vorzugsweise 1: (1,7 bis 2,3):(10 bis 15) gearbeitet. Der Reaktionsdruck liegt üblicherweise im Bereich von 1 bis 3 bar und die Gesamtraumbelastung beträgt vorzugsweise 7.500 bis 2900 N1/1/h. Die Propenlast beträgt typisch 90 bis 160 N1/1/h oder auch bis 200 N1/1/h und mehr.

Bevorzugt wird der Einzonen-Vielkontaktrohr-Festbettreaktor mit dem Beschickungsgasgemisch von oben angeströmt. Als Wärmeaustauschmittel wird zweckmäßig eine Salzschmelze, bevorzugt bestehend aus 60 Gew.-% Kaliumnitrat (KNO₃) und 40 Gew.-% Natriumnitrit (NaNO₂), eingesetzt.

Über den Reaktor betrachtet können Salzschmelze und Reaktionsgasgemisch sowohl im Gleichstrom als auch im Gegenstrom geführt werden. Die Salzschmelze selbst wird bevorzugt mäanderformig um die Kontaktrohre geführt.

Strömt man die Kontaktrohre von oben nach unten an, ist es zweckmäßig, die Kontaktrohre von unten nach oben wie folgt zu beschikken (würde man von unten nach oben anströmen, würde man die Beschickungsabfolge in zweckmäßiger Weise umkehren):
- zunächst auf einer Länge von 40 bis 60 % der Kontaktrohrlänge entweder nur Katalysator, oder ein Gemisch aus Katalysator und Inertmaterial, wobei letzteres, bezogen auf die Mischung, ein Gewichtsanteil von bis zu 20 Gew.-% ausmacht (Abschnitt C);
- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 40 Ges.-% ausmacht (Abschnitt B); und
- abschließend auf einer Länge von 10 bis 20 % der Gesamtrohrlänge eine Schüttung aus Inertmaterial (Abschnitt A).

Bevorzugt ist der Abschnitt C unverdünnt.

Vorgenannte Beschickungsvariante ist insbesondere dann zweckmäßig, wenn als Katalysatoren solche gemäß Beispiel 1 der DE-A 10046957 oder gemäß Beispiel 3 der DE-A 10046957 und als Inertmaterial Ringe aus Steatit mit der Geometrie 7 mm x 7 mm x 4 mm (Außendurchmesser x Höhe x Innendurchmesser) verwendet werden. Hinsichtlich der Salzbadtemperatur gilt das in der DE-A 4431957 Gesagte.

Die Durchführung des ersten Schrittes, vom Propylen zum Acrolein, kann mit den beschriebenen Katalysatoren aber auch z. B. in einem Zweizonen-Vielkontaktrohr-Festbettreaktor durchgeführt werden, wie es die DE-A 19910506 beschreibt. In beiden vorstehend beschriebenen Fällen liegt der erzielte propenumsatz bei einfachem Durchgang normalerweise bei Werten ≥ 90 mol-%, bzw. ≥ 95 mol-%. Die Durchführung des zweiten Schrittes, vom Acrolein zur Acryl säure, kann mit den beschriebenen Katalysatoren z. B. in einem Einzonen-Vielkontaktrohr-Festbettreaktor durchgeführt werden, wie es die DE-A 4431949 beschreibt.

Zur Erzeugung des Beschickungsgasgemisches kann dabei von Roh-Acrolein ausgegangen werden. Besonders vorteilhaft ist es aber, zur Beschickung das Acrolein enthaltende Produktgasgemisch einer auf den ersten Schritt gerichteten ersten Stufe (wie sie vorstehend beschrieben wurden) zu verwenden (gegebenenfalls nach erfolgter Zwischenkühlung desselben). Der für den zweiten Schritt benötigte Sauerstoff wird dabei bevorzugt als Luft zugesetzt und im zweiten Fall dem Produktgasgemisch unmittelbar zugegeben.

In der Regel ist das Beschickungsgasgemisch einer solchen zweiten Stufe dann wie folgt zusammengesetzt: Acrolein : Sauerstoff : Wasserdampf : Inertgas-Volumerlverhältnis (N1) von 1:(1 bis 3):(0 bis 20):(3 bis 30), vorzugsweise von 1:(1 bis 3):(0,5 bis 10):(7 bis 18).

Der Reaktionsdruck beträgt in der Regel auch hier 1 bis 3 bar und die Gesamtraumbelastung liegt vorzugsweise bei 1000 bis 2500 Nl/ 1/h. Die Acroleinlast beträgt typisch 80 bis 150 N1/1/h oder auch bis 180 N1/1/h und mehr.

Bevorzugt wird der Einzonen-Vielkontaktrohr-Festbettreaktor mit dem Beschickungsgasgemisch ebenfalls von oben angeströmt. Als Wärmeaustauschmittel wird auch in der zweiten Stufe in zweckmäßiger Weise eine Salzschmelze, bevorzugt aus 60 Gew.-% Kaliumnitrat (KNO₃) und 40 Gew.-% Natriumnitrit (NaNO₂) bestehend, eingesetzt. Über den Reaktor betrachtet können Salzschmelze und Reaktionsgasgemisch sowohl im Gleichstrom als auch im Gegenstrom geführt werden. Die Salzschmelze selbst wird bevorzugt mäanderförmig in die Kontaktrohre geführt.

Strömt man die Kontaktrohre von oben nach unten an, ist es zweckmäßig, die Kontaktrohre von unten nach oben wie folgt zu beschikken:
- zunächst auf einer Länge von 50 bis 70 % der Kontaktrohrlänge entweder nur Katalysator oder ein Gemisch aus Katalysator und Inertmaterial, wobei letzteres, bezogen auf die Mischung, einen Gewichtsanteil von bis zu 20 Gew.-% ausmacht (Abschnitt C) ;
- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 40 Gew.-% ausmacht (Abschnitt B); und
- abschließend auf einer Länge von 5 bis 20 % der Gesamtrohrlänge eine Schüttung aus Inertmaterial (Abschnitt A).

Bevorzugt ist der Abschnitt C unverdünnt.

Vorgenannte Beschickungsvariante ist insbesondere dann zweckmäßig, wenn als Katalysatoren solche gemäß Herstellungsbeispiel 5 der DE-A 10046928 oder solche gemäß DE-A 19815281 und als Inertmaterial Ringe aus Steatit mit der Geometrie 7 mm x 7 mm x 4 mm oder 7 mm x 7 mm x 3 mm (jeweils Außendurchmesser x Höhe x Innendurchmessser) verwendet werden. Hinsichtlich der Salzbadtemperatur gilt das in der DE-A 44 319 49 Gesagte. Sie wird in der Regel so gewählt, daß der erzielte Acroleinumsatz bei einfachem Durchgang normalerweise ≥ 90 mol-%, bzw. ≥ 95 mol-% beträgt.

Die Durchführung des zweiten Schrittes, vom Acrolein zur Acrylsäure, kann mit den beschriebenen Katalysatoren aber auch z. B. in einem Zweizonen-Vielkontaktrohr-Festbettreaktor durchgeführt werden, wie es die DE-19910508 beschreibt. Für den Acroleinumsatz gilt das oben Genannte. Auch im Fall der Durchführung des zweiten Schrittes in einem Zweizonen-Vielkontaktrohr-Festbettreaktor kann zur Erzeugung des Beschickungsgasgemisches entweder von Roh-Acrolein ausgegangen werden, oder unmittelbar das Produktgasgemisch einer auf den ersten Schritt gerichteten ersten Stufe (gegebenenfalls nach erfolgter Zwischenkühlung desselben) verwendet werden (wie sie vorstehend beschrieben wurde). Der für den zweiten Schritt benötigte Sauerstoff wird dabei bevorzugt als Luft zugesetzt und im zweiten Fall dem Produktgasgemisch unmittelbar zugegeben.

Bei einer zweistufigen Fahrweise mit unmittelbarer Weiterverwendung des Produktgasgemisches der ersten Stufe zur Beschickung der zweiten Stufe, wird man in der Regel zwei Einzonen-Vielkontaktrohr-Festbettreaktoren oder zwei Zweizonen-Vielkontaktrohr-Festbettreaktoren hintereinanderschalten. Eine gemischte Hintereinanderschaltung (Einzonen/Zweizonen oder umgekehrt) ist auch möglich.

Zwischen den Reaktoren kann sich ein Zwischenkühler befinden, der gegebenenfalls Inertschüttungen enthalten kann, die eine Filterfunktion ausüben können.

Die Salzbadtemperatur von Vielkontaktrohrreaktoren für die erste Stufe beträgt in der Regel 300 bis 400°C. Die Salzbadtemperatur von Vielkontaktrohrreaktoren für die zweite Stufe beträgt meist 200 bis 350°C.

Erfindungswesentlich ist, daß für alle vorgenannten Konstellationen das erfindungsgemäße Verfahren sowohl dann angewendet werden kann, wenn die beiden Stufen voneinander unabhängig betrieben werden, als auch wenn sie wie vorstehend dargestellt in Hintereinanderschaltung betrieben werden. Es ist aber auch dann erfolgreich, wenn beide Schritte wie in der DE-A 10121592 beschrieben in einem Reaktor an einer Beschickung vollzogen werden.

Dabei kann sich der Teilkatalysatorwechsel in allen Fällen in Strömungsrichtung ganz besonders bevorzugt auf 35 bis 45 % der Schüttlänge des Katalysatorfestbettes erstrecken (eine zu 100 % aus Inertmaterial bestehende Deckbeschikkung (die Erstbeschickung aus der Srömungssicht) wird dabei nicht zum Katalysatorfestbett zugehörig betrachtet; aus Gründen der Zweckmäßigkeit würde diese Deckbeschickung aber mit ausgetauscht; in entsprechender Weise würde auch eine zu 100 % aus Inertmaterial bestehende Abschlußbeschickung (die Endbeschickung aus der Strömungssicht) dabei nicht zum Katalysatorfestbett zugehörig betrachtet; eine zu 100 % aus inertmaterial bestehende Zwischenbeschickung würde jedoch zum Katalysatorfestbett zugehörig betrachtet).

Abschließend sei noch erwähnt, daß ein Teil des Beschickungsgasgemisches der ersten Stufe ("Propen-Acrolein") sogenanntes Kreisgas sein kann. Hierbei handelt es sich um Gas, das nach der Produktabtrennung (Acrylsäureabtrennung) vom Produktgasgemisch der zweiten Stufe verbleibt und bei einer Hintereinanderschaltung der beiden Stufen in der Regel zum Teil als inertes Verdünnungsgas zum Beschicken der ersten und/oder zweiten Stufe rückgeführt wird.

Eine typische Kreisgaszusammensetzung lautet:
- 0: - 0,1 vol-% sonstige, z. B. Diphenyl, Diphenylether und/oder Dimenthylphthalat,
- 0: - 0, 1 Vol-% Acrylsäure,
- 0: - 0,1 Vol-% Acrolein,
- 3: - 5 % Vol-% Sauerstoff,
- 1: - 5 Vol.-% Wasserdampf,
- 0: - 3 Vol.-% Kohlenmonoxid,
- 0: - 8 Vol.-% Kohlendioxid,
- 0: - 2 Vol.-% Propan,
- 0,1: - 0,5 Vol-% Propylen,
- 85: - 95 Vol.-% Stickstoff.

Dabei kann die Acrylsäureabtrennung wie in der EP-A 982 287, der EP-A 982 289, der DE-A 19924532, der DE-A 10115277, der DE-A 19606877, der DE-A 19740252, der DE-A 19627847, der DE-A 10053086, der EP-A 982 288 und der DE-A 19627847 beschrieben abgetrennt werden.

Prinzipiell kann der Teilkatalysatorwechsel zu jedem Zeitpunkt, d. h. z. B. nach einjähriger, zweijähriger, dreijähriger oder mehrjähriger Betriebsdauer durchgeführt werden. In der Regel wird man ihn gemäß Wirtschaftlichkeitsüberlegungen durchführen.

Abschließend sei erwähnt, daß sich der erfindungsgemäße Teilkatalysatorwechsel in der Regel auch vorteilhaft auf den Druckverlust beim Durchgang des Reaktionsgemisches durch die Katalysatorbeschickung auswirkt.

Außerdem werden die Wärmeaustauschmittel (Salzschmelzen) normalerweise in solchen Mengen durch die relevanten Vielkontaktrohr-Festbettreaktoren geführt, daß der Unterschied zwischen ihrer Eingangs- und ihrer Ausgangstemperatur in der Regel ≤ 5°C beträgt.

### Beispiele und Vergleichsbeispielen

### A) Verfahren der zweistufigen heterogen katalysierten Gasphasen-Partialoxidation von Propylen zu Acrylsäure in zwei hintereinandergeschalteten Einzonen-Vielkontaktrohr-Festbettreaktoren

### I. Beschreibung der allgemeinen Verfahrensbedingungen in der ersten Stufe

- Verwendetes Wärmeaustauschmittel:: Salzschmelze, bestehend aus 60 Gew.-% Kaliumnitrat und 40 Gew.-% Natriumnitrit.
- Material der Kontaktrohre:: ferritischer Stahl.
- Abmessungen der Kontaktrohre:: 3200 mm Länge; 25 mm Innendurchmesser; 30 mm Außendurchmesser (Wandstärke: 2,5 mm).
- Anzahl der Kontaktrohre im Rohrbündel:: 25500.

- Reaktor:: Zylinderförmiger Behälter eines Durchmessers von 6800 mm; ringförmig angeordnetes Rohrbündel mit einem freien zentralen Raum.
Durchmesser des zentralen freien Raumes: 1000 mm. Abstand der am weitesten außen liegenden Kontaktrohre zur Behälterwand: 150 mm. Homogene Kontaktrohrverteilung im Rohrbündel (6 äquidistante Nachbarrohre pro Kontaktrohr).
Kontaktrohrteilung: 38 mm.
Die Kontaktrohre waren mit ihren Enden in Kontaktrohrböden der Dicke 125 mm abdichtend befestigt und mündeten mit ihren Öffnungen in je eine am oberen bzw. unteren Ende mit dem Behälter verbundenen Haube.
Zuführung des Wärmeaustauschmittels zum Rohrbündel:
Das Rohrbündel war durch drei zwischen den Kontaktrohrböden längs derselben aufeinanderfolgend angebrachte Umlenkscheiben (Dicke jeweils 10 mm) in 4 äquidistante (jeweils 730 mm) Längsabschnitte (Zonen) geteilt.

Die unterste und die oberste Umlenkscheibe wies Ringgeometrie auf, wobei der Ringinnendurchmesser 1000 mm betrug und der Ringaußendurchmesser sich bis zur Behälterwand abdichtend erstreckte. Die Kontaktrohre waren an den Umlenkscheiben nicht abdichtend befestigt. Vielmehr waren eine Spaltbreite < 0,5 mm aufweisende Spalte so belassen, daß die Querströmungsgeschwindigkeit der Salzschmelze innerhalb einer Zone möglichst konstant war.

Die mittlere Umlenkscheibe war kreisförmig und erstreckte sich bis zu den am weitesten außen liegenden Kontaktrohren des Rohrbündels.

Die Kreisführung der Salzschmelze wurde durch zwei Salzpumpen bewerkstelligt, von denen jede eine Rohrbündellängshälfte versorgte.

Die Pumpen drücken die Salzschmelze in einen um den Reaktormantel unten angebrachten Ringkanal, der die Salzschmelze über den Behälterumfang verteilte. Durch im Reaktormantel befindliche Fenster gelangte die Salzschmelze im untersten Längsabschnitt zum Rohrbündel. Die Salzschmelze floß dann der Vorgabe der Umlenkbleche folgend in der Abfolge
- von außen nach innen,
- von innen nach außen,
- von außen nach innen,
- von innen nach außen,
im wesentlichen mäanderformig, über den Behälter betrachtet, von unten nach oben. Durch im obersten Längsabschnitt um den Behälterumfang angebrachte Fenster sammelte sich die Salzschmelze in einem oberen um den Reaktormantel angebrachten Ringkanal und wurde nach Abkühlung auf die ursprüngliche Eintrittstemperatur durch die Pumpen wieder in den unteren Ringkanal gedrückt.

Zusammensetzung des Reaktionsgasausgangsgemisches (Gemisch aus Luft, Polymer grade Propylen und Kreisgas):
5,4 Vol.-% Propen,
10,5 Vol.-% Sauerstoff,
1,2 Vol.-% COₓ,
81,3 Vol.-% N₂,
1,6 Vol.-% H₂O.

- Reaktorbeschickung:: Salzschmelze und Reaktionsgasgemisch wurden über den Reaktor betrachtet im Gegenstrom geführt. Die Salzschmelze trat unten ein, das Reaktionsgasgemisch oben.
Die Eintrittstemperatur der Salzschmelze betrug am Anfang 337°C. Die Austrittstemperatur der Salzschmelze lag bei 339°C.
Die Pumpleistung betrug 6200 m³ Salzschmelze/h.
Das Reaktionsgasausgangsgemisch wurde dem Reaktor mit einer Temperatur von 300°C zugeführt.
- Belastung mit Reaktionsgasausgangsgemisch:: 68845 Nm³/h.
- Proponlast der Katalysatorbeschickung:: 110 h⁻¹.
- Kontaktrohrbeschickung (von oben nach unten):: Zone A: 50 cm
Vorschüttung aus Steatit-Ringen der Geometrie 7 mm x 7 mm x 4 mm (Außendurchmesser x Länge x Innendurchmesser)
Zone B: 100 cm
Katalysatorbeschickung mit einem homogenen Gemisch aus 30 Gew.-% an Steatit-Ringen der Geometrie 5 mm x 3 mm x 2 mm (Außendurchmesser x Länge x Innendurchmesser) und 70 Gew.-% Vollkatalysator aus Zone C).
Zone C: 170 cm
Katalysatorbeschickung mit ringförmigem (5 mm x 3 mm x 2 mm = Außendurchmesser x Länge x Innendurchmesser) Vollkatalysator gemäß Beispiel 1 der DE-A 10046957.

### II. Beschreibung der Zwischenkühlung

Das die erste Reaktionsstufe mit einer Temperatur von 339°C verlassende Produktgasgemisch wurde zum Zweck der Zwischenkühlung durch einen mit einer Salzschmelze aus 60 Gew.-% Kaliumnitrat und 40 Gew.-% Natriumnitrit gekühlten Einzonen-Rohrbündelwärmetauscher aus ferritischem Stahl geführt, der unmittelbar an den Reaktor angeflanscht war. Der Abstand des unteren Rohrbodens des Reaktors zum oberen Rohrboden des Kühlers betrug dabei 10 cm. Die Salzschmelze und das Produktgasgemisch wurden dabei über den Wärmetauscher betrachtet im Gegenstrom geführt. Das Salzbad selbst floß in gleicher Weise wie im Einzonen-VielkontaktrohrFestbettreaktor mäanderförmig mm die Kühlrohre, durch die das Produktgasgemisch geleitet wurde. Die Länge der Kühlrohre betrug 1,65 m, ihr Innendurchmesser lag bei 2,6 cm und ihre Wanddicke war 2,5 mm. Die Anzahl der Kühlrohre belief sich auf 8000. Der Durchmesser des Wärmetauschers betrug 7,2 m.

Sie waren mit einheitlicher Rohrteilung gleichmäßig über den Querschnitt verteilt.

In den Eingang der Kühlrohre (in Strömungsrichtung) waren Spiralen aus Edelstahl eingeführt, deren Querschnitt nahezu dem der Kühlrohre entsprach. Sie dienten der Verbesserung des Wärmeübergangs.

Das Produktgasgemisch verließ den Zwischenkühler mit einer Temperatur von 250°C. Anschließend wurde ihm komprimierte Luft, die eine Temperatur von 140°C aufwies, in einer Menge von 6692 Nm³/h zugemischt.

Das dabei erhaltene Beschickungsgasgemisch wurde mit einer Temperatur von 220°C dem Einzonen-Vielkontaktrohr-Festbettreaktor der zweiten Stufe zugeführt.

### III. Beschreibung der allgemeinen Verfahrensbedingungen in der zweiten Stufe

Es wurde ein Einzonen-Vielkontaktrohr-Festbettreaktor verwendet, der mit jenem der ersten Stufe baugleich war.

Salzschmelze und Reaktionsgasgemisch wurden über den Reaktor betrachtet im Gegenstrom geführt. Die Salzschmelze trat unten ein, das Reaktionsgasgemisch oben.

Die Eintrittstemperatur der Salzschmelze betrug am Anfang 265°C. Die Austrittstemperatur der Salzschmelze lag bei 267°C. Die Pumpleistung betrug 6200 m³ Salzschmelze/h.

Die Belastung mit Beschickungsgasgemisch lag bei 75537 Nm³/h.
- Die Kontaktrohrbeschickung (von oben nach unten) war:: Zone A:
20 cm Vorschüttung aus Steatit-Ringen der Geometrie 7 mm x 7 mm x 4 mm (Außendurchmesser x Länge x Innendurchmesser).
Zone B:
100 cm Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysatoz aus Zone C.
Zone C:
200 cm Katalysatorbeschickung mit ringförmigem (ca. 7 mm x 3 mm x 4 mm) Schalenkatalysator gemäß Herstellungsbeispiel 5 der DE-A 10046928.

Die Analyse des Produktgasgemisches der zweiten Stufe ergab folgende Ergebnisse:
Der Umsatz des in der ersten Stufe gebildeten Acrolein betrug, mit frisch formierter Katalysatorbeschickung der zweiten Stufe, bei einer Eintrittstemperatur der Salzschmelze in die zweite Stufe von 265°C 99,3 mol-% bei einer Selektivität der Acrylsäurebildung von 88,9 mol-% (diese Angaben sind ebenso wie die nachfolgenden Angaben stets auf einfachen Durchgang bezogen).

Mit zunehmender Betriebsdauer fiel der Acroleinumsatz in der zweiten Stufe.

Durch sukzessives Erhöhen der Eintrittstemperatur der Salzschmelze in die zweite Stufe konnte dieser Aktivitätsverlust ausgeglichen werden.

Nach mehrjähriger Betriebsdauer unter ansonsten identischen Bedingungen, betrug die dazu erforderliche Eintrittstemperatur der Salzschmelze in die zweite Stufe 290°C. Die Selektivität der Acrylsäurebildung sank gleichzeitig auf 87, 5 mol-%.

Absaugen der Zone A und von 30 cm der Zone B und Ersatz des Abgesaugten durch entsprechende Frischbeschickung in der zweiten Stufe führte bereits bei einer Eintrittstemperatur Salzschmelze in die zweite Stufe von 287°C zum ursprünglichen Acroleinumsatz, bei einer Selektivität der Acrylsäurebildung von 87,9 mol-% (Vergleichsbeispiel 1).

Absaugen der Zone A und von 60 cm der Zone B und Ersatz des Abgesaugten durch entsprechende Frischbeschickung in der zweiten Stufe führte bereits bei einer Eintrittstemperatur der Salzschmelze in die zweite Stufe von 281°C zum ursprünglichen Acroleinumsatz, bei einer Selektivität der Acrylsäurebildung von 88,3 mol-% (Vergleichsbeispiel 2).

Absaugen der Zone A, der Zone B und von 20 cm der Zone C und Ersatz des Abgesaugten durch entsprechende Frischbeschickung in der zweiten Stufe führte bereits bei einer Eintrittstemperatur der Salzschmelze in die zweite Stufe von 270°C zum ursprünglichen Acroleinumsatz, bei einer Selektivität der Acrylsäurebildung von 88,9 mol-% (Beispiel 1).

Absaugen der Zone A, der Zone B und von 50 cm der Zone C und Ersatz des Abgesaugten durch entsprechende Frischbeschickung in der zweiten Stufe führte bereits bei einer Eintrittstemperatur der Salzschmelze in die zweite Stufe von 267°C zum ursprünglichen Acroleinumsatz, bei einer Selektivität der Acrylsäurebildung von 88,9 mol-% (Beispiel 2).

D. h., ein Austausch von weniger als 50 % des Aktivmassenkatalysators der zweiten Stufe führte zu einer nahezu 100%igen Wiederherstellung von Aktivität und Selektivität der Katalysatorbeschickung der zweiten Stufe.

Die Abtrennung der Acrylsäure und die Kreisgasbildung wurden wie in der WO 97/48669 beschrieben vorgenommen.
- B): Das vorgenannte Beispiel A) führt qualitativ zum selben Ergebnis, wenn anstelle der Einzonen-Vielkontaktrohr-Festbettreaktoren die Zweizonen-Vielkontaktrohr-Fesbestreaktoren gemäß der DE-A 19910506 und der DE-A 19910508 und die in diesen Schriften genannten Verfahrensbedingungen angewendet werden.
- C): Sowohl in Beispiel A) als auch in Beispiel B) kann als Erststufenkatalysator auch der Katalysator gemäß Beispiel 3 der DE-A 10046957 und als Zweitstufenkatalysator ein Katalysator gemäß der DE-A 19815281 verwendet werden. Das volumenspezifische Aktivitätsprofil wird dabei beibehalten.

## Patentansprüche

1. Verfahren der heterogen katalysierten Gasphasen-Partialoxidation wenigstens einer organischen Verbindung mit molekularem Sauerstoff an einem in einem Vielkontaktrohr-Festbettreaktor befindlichen Katalysatorfestbett, bei dem ab einem bestimmten Betriebszeitpunkt mit zunehmender Betriebsdauer eine zunehmende Minderung der Qualität der Katalysatorbeschickung einhergeht, **dadurch gekennzeichnet, daß** zur Rückgewinnung der Qualität der Katalysatorbeschickung nicht die gesamte gebrauchte Katalysatorbeschickung sondern nur eine Teilmenge derselben aus dem Vielkontaktrohr-Festbettreaktor entnommen und durch eine frische Katalysatorbeschickung ersetzt wird, und
- die Gasphasen-Partialoxidation diejenige von Acrolein zu Acrylsäure ist;
- die volumenspezifische Aktivität der Katalysatorbeschickung in Strömungsrichtung des Reaktionsgasgemischs kontinuierlich oder stufenförmig zunimmt; und
- sich der Teilkatalysatorwechsel in Strömungsrichtung auf 30 bis 50-% der Schüttlänge des Katalysatorfestbetts erstreckt.

2. Verfahren nach Anspruch 1, **dadurch gekennzeichnet, daß** die Gasphasen-Partialoxidation die zweite Stufe einer zweistufigen Gasphasen-Partialoxidation ist.

3. Verfahren nach Anspruch 1 oder 2, **dadurch gekennzeichnet, daß** die Gasphasen-Partialoxidation die Partialoxidation von Acrolein zu Acrylsäure in einer zweistufigen Gasphasen-Partialoxidation von Propylen zu Acrylsäure ist.

4. Verfahren nach Anspruche 2 oder 3, **dadurch gekennzeichnet, daß** das Produktgasgemisch der ersten Stufe als solches zur Beschickung der zweiten Stufe verwendet wird.

## Claims

1. A process for the heterogeneously catalyzed gas-phase partial oxidation of at least one organic compound with molecular oxygen over a fixed catalyst bed present in a fixed-bed reactor containing a plurality of catalyst tubes, in which there is an increasing reduction of the quality of the catalyst load with increasing duration of operation from a specific operating time, wherein, in order to recover the quality of the catalyst load, only a portion of spent catalyst load, instead of the entire spent catalyst load, is removed from the fixed-bed reactor containing a plurality of catalyst tubes and is replaced by a fresh catalyst load, and
- the gas-phase partial oxidation is that of acrolein to acrylic acid;
- the volume-specific activity of the catalyst load in the direction of flow of the reaction gas mixture increases continuously or stepwise; and
- the partial catalyst exchange in the direction of flow extends to 30 to 50% of the length of the fixed catalyst bed.

2. The process according to claim 1, wherein the gas-phase partial oxidation is the second stage of a two-stage gas-phase partial oxidation.

3. The process according to claim 1 or 2, wherein the gas-phase partial oxidation is the partial oxidation of acrolein to acrylic acid in a two-stage gas-phase partial oxidation of propylene to acrylic acid.

4. The process according to claim 2 or 3, wherein the product gas mixture of the first stage is used as such for feeding the second stage.

## Revendications

1. Procédé d'oxydation partielle en phase gazeuse sous catalyse hétérogène d'au moins un composé organique avec de l'oxygène moléculaire sur un lit catalytique fixe se trouvant dans un réacteur à lit fixe et à tubes de contact multiples, selon lequel à partir d'un certain moment de l'exploitation, la durée d'exploitation croissante s'accompagne d'une diminution croissante de la qualité du revêtement catalytique, **caractérisé en ce que**, pour récupérer la qualité du revêtement catalytique, seule une partie du revêtement catalytique usagé, et non la totalité, est extraite du réacteur à lit fixe et à tubes de contact multiples, et remplacée par un revêtement catalytique frais, et
- l'oxydation partielle en phase gazeuse est celle d'acroléine en acide acrylique ;
- l'activité spécifique au volume du revêtement catalytique augmente continuellement ou graduellement dans la direction de l'écoulement du mélange gazeux réactionnel ; et
- le remplacement partiel du catalyseur dans la direction d l'écoulement s'étend sur 30 à 50 % de la longueur de garnissage du lit catalytique fixe.

2. Procédé selon la revendication 1, **caractérisé en ce que** l'oxydation partielle en phase gazeuse est la seconde étape d'une oxydation partielle en phase gazeuse à deux étapes.

3. Procédé selon la revendication 1 ou 2, **caractérisé en ce que** l'oxydation partielle en phase gazeuse est l'oxydation partielle d'acroléine en acide acrylique lors d'une oxydation partielle en phase gazeuse à deux étapes de propylène en acide acrylique.

4. Procédé selon la revendication 2 ou 3, **caractérisé en ce que** le mélange gazeux de produits de la première étape est utilisé en tant que tel pour l'alimentation de la seconde étape.
